# EUROPÄISCHE PATENTSCHRIFT

(11) **EP 1 868 975 B1**
(45) Veröffentlichungstag und Bekanntmachung des Hinweises auf die Patenterteilung: **13.06.2012**
(21) Anmeldenummer: 06725582.8
(22) Anmeldetag: 05.04.2006
(51) Int. Cl.: C07C 37/11, C07C 39/15, C07F 9/02

(54) **VERBESSERTES VERFAHREN ZUR HERSTELLUNG VON BIPHENOLEN AUS MONOPHENOLEN**
IMPROVED METHOD FOR PRODUCING BIPHENOLS FROM MONOPHENOLS
PROCEDE AMELIORE DE PRODUCTION DE BIPHENOLS A PARTIR DE MONOPHENOLS

(30) Priorität: 06.04.2005 DE 102005015893
(43) Veröffentlichungstag der Anmeldung: 26.12.2007
(73) Patentinhaber: BASF SE, 67056 Ludwigshafen (DE)
(72) Erfinder: BARTSCH, Michael, 67433 Neustadt (DE); HADERLEIN, Gerd, 67269 Grünstadt (DE); AECHTNER, Tobias, 68165 Mannheim (DE); SCHEIDEL, Jens, 69493 Hirschberg (DE); DIENES, Christian, 76829 Landau (DE); TEMPEL, Alexandre, 67549 Worms (DE); HOFRICHTER, Thorsten, 67227 Frankenthal (DE); WEINLE, Werner, 74177, Bad Friedrichshall (DE)
(86) Internationale Anmeldenummer: PCT/EP2006/061352
(87) Internationale Veröffentlichungsnummer: WO 2006/106123

(56) Entgegenhaltungen:
- WO-A-2004/069779
- WO-A2-2004/076464
- US-A- 6 077 979

## Beschreibung

Die Erfindung betrifft ein Verfahren zur Herstellung von Biphenolen der allgemeinen Formel I 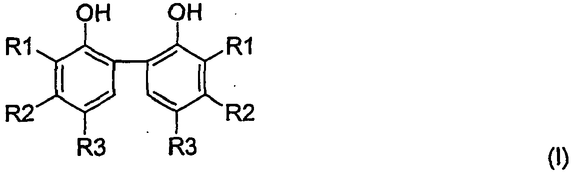 durch Umsetzung von Monophenolen der allgemeinen Formel II 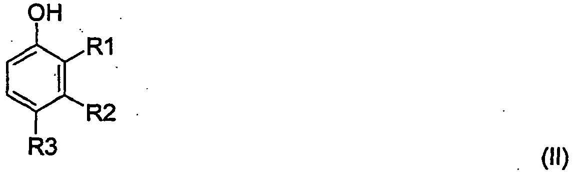 wobei die Reste R1, R2 und R3 unabhängig voneinander Wasserstoff, Alkyl, Aryl oder Arylalkyl mit 1 bis 10 C-Atomen bedeuten, in Gegenwart eines Oxidationsmittels in einem Reaktor, dadurch gekennzeichnet, dass
a) der Reaktor keine als Stromstörer wirkenden stationären Einbauten enthält,
b) pro ein Mol Monophenol insgesamt maximal 0,6 Mol Oxidationsmittel eingesetzt werden, und
c) das Oxidationsmittel innerhalb einer Zeitspanne von 10 min bis 24 Stunden entweder kontinuierlich, oder diskontinuierlich in mehreren Portionen, zudosiert wird, wobei die pro Zeiteinheit zudosierte Oxidationsmittelmenge nicht über die gesamte Zeitspanne konstant ist, sondern variiert wird.

Unsubstituierte und substituierte Biphenole können vielfältig verwendet werden; beispielsweise als Zwischenprodukte in chemischen Synthesen oder als Monomere zur Polymerherstellung. Auch zur Herstellung von Katalysatorsystemen für die Hydocyanierung von Pentennitrilen zu Adipodinitril werden Biphenole verwendet: das Katalysatorsystem enthält üblicherweise Komplexverbindungen aus Nickel(0) und phosphorhaltigen Chelatliganden, und die Biphenole sind Ausgangsstoff bei der Herstellung dieser Chelatliganden, siehe z.B. die US-PS 5 981 772. Die genannte Hydrocyanierung wird großtechnisch durchgeführt und erfordert erhebliche Katalysatormengen, weshalb auch die Biphenole in großtechnischen Verfahren hergestellt werden.

Die Biphenole werden üblicherweise durch oxidative Kupplung entsprechender Monophenole hergestellt, wobei die Ausbeute dieser Synthesen verbesserungswürdig ist. Die WO-A 03/045883 beschreibt die Herstellung bestimmter substituierter Biphenole mit einem kupferhaltigen Katalysator.

In der US-PS 6 077 979 wird ein Verfahren zur Herstellung von 3,3',5,5'-Tetramethyl-2,2'-biphenol durch Umsetzung von 2,4-Dimethylphenol (DMP) mit einem Oxidationsmittel (Persulfat oder Wasserstoffperoxid) und einem Eisenkatalysator bei 0 bis 100°C. Das molare Verhältnis DMP : Oxidationsmittel beträgt 1,2 : 1 bis 1 : 1,2, ist also ungefähr äquimolar. Ausweislich der Beispiele betragen die eingesetzten Mengen an Reaktionspartnern und Lösungsmitteln maximal einige hundert Gramm bzw. wenige Liter (Example 1); der größte verwendete Reaktor ist ein gerührter 5 Liter-Kolben. Das Oxidationsmittel wird entweder auf einmal zu Reaktionsbeginn oder in Example 1 gleichförmig innerhalb von 4 Stunden zugefügt. Wo 2004/069775 offenbart ebenfalls ein Verfahren zur Herstellung vom Biphenolen.

Der Anmelder der vorliegenden Patentanmeldung hat das Verfahren der US-PS 6 077 979 in einen großtechnischen Maßstab übertragen. Dazu wurde ein 2 m³-Rührkessel verwendet, worin wie allgemein üblich Stromstörer zur besseren Durchmischung des Kesselinhalts angebracht waren. Dabei traten unerwartet folgende Probleme auf:
- Die gebildeten Reaktionsprodukte (Phenole) verklebten mit dem Rührer, wodurch eine Unwucht entstand. Um eine Zerstörung des Rührers zu vermeiden, musste er häufig gereinigt werden. Diese Produktionsunterbrechung zwecks Reinigung verschlechterte die Wirtschaftlichkeit des Verfahrens erheblich.
- Im Bereich hinter den Stromstörern lagerten sich gebildete Phenole ab. Die Produktion musste unterbrochen und der Kessel gereinigt werden.
- Es wurden erhebliche Mengen unerwünschter Nebenprodukte, insbesondere oligomere Phenolverbindungen, gebildet. Die in der US-PS 6 077 979 für den 5 Liter-Ansatz genannte Reinheit von 95 % wurde erst nach zweimaligem Umkristallisieren erreicht und die Ausbeute war deutlich geringer als in der US-PS angegeben.
- Diese Nebenprodukte erschwerten die Aufarbeitung der Reaktionsmischung erheblich, da sich das gewünschte Biphenol nicht durch Filtration oder andere fest/flüssig-Trennverfahren abtrennen ließ. Auch durch Kristallisation ließen sich die Nebenprodukte nur unzureichend vom Wertprodukt Biphenol abtrennen. Dies verminderte die Wirtschaftlichkeit des Verfahrens nochmals.

Es bestand die Aufgabe, den geschilderten Nachteilen abzuhelfen. Es sollte ein Verfahren zur Herstellung von Biphenolen aus Monophenolen bereit gestellt werden, das auch großtechnisch problemlos durchgeführt werden kann. Insbesondere sollten sich keine Ablagerungen oder klebrige Anhaftungen am Reaktor bzw. dessen Rührorganen bilden. Der Reaktor sollte nur selten zwecks Reinigung abgestellt werden müssen.

Weiterhin sollte das gewünschte Biphenol in hoher Reinheit anfallen, und in einfacher Weise, z.B. durch Filtration aus der Reaktionsmischung abtrennbar sein. Schließlich sollten weniger unerwünschte oligomere Phenole bzw. andere Nebenprodukte gebildet werden.

Demgemäß wurde das eingangs definierte Verfahren gefunden. Außerdem wurden die damit erhältlichen Biphenole und deren genannte Verwendung gefunden. Bevorzugte Ausführungsformen der Erfindung sind den Unteransprüchen zu entnehmen.

Als Monophenole eignen sich solche der allgemeinen Formel II 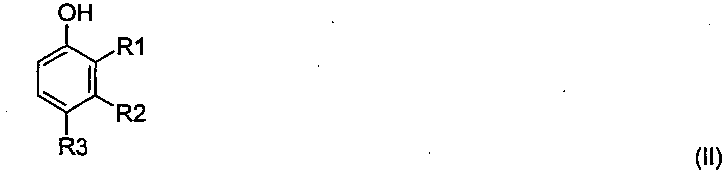 wobei die Reste R1, R2 und R3 - nachfolgend zusammenfassend als R bezeichnet - unabhängig voneinander Wasserstoff, Alkyl, Aryl oder Arylalkyl mit 1 bis 10 C-Atomen bedeuten. Alkyl schließt Cycloalkyl ein. Die Reste R können außerdem auch Heteroatome wie Halogen, O, N, P, S oder Si enthalten.

Bevorzugt enthalten die Reste R 1 bis 8, insbesondere 1 bis 6 C-Atome. Besonders bevorzugt ist R gleich Alkyl, vorzugsweise C₁₋₄-Alkyl, insbesondere n- oder iso-Propyl, Ethyl oder Methyl. Methyl ist ganz besonders bevorzugt.

Bevorzugt ist einer der drei Reste R1, R2 und R3 gleich Wasserstoff, d.h. bevorzugt sind die Monophenole zweifach mit Resten R substituiert. Von den drei Substitutionsmustern 3,4 (also R1 gleich H), 2,4 (R2 gleich H) und 2,3 (R3 gleich H) sind die 2,4-substituierten Monophenole (R2 gleich H) bevorzugt.

In einer bevorzugten Ausführungsform bedeuten zwei der Reste R1, R2 und R3 Methyl und ein Rest Wasserstoff. Insbesondere sind R1 und R3 gleich Methyl und R2 gleich Wasserstoff, d.h. bevorzugt ist das Monophenol 2,4-Dimethylphenol und das daraus erhaltene Biphenol ist 3,3',5,5'-Tetramethyl-2,2'-biphenol.

Die genannten Monophenole sind in an sich bekannter Weise durch Alkylierung bzw. Arylierung von Phenol mit Alkenen, Alkoholen oder Alkyl- bzw. Arylhalogeniden erhältlich, oder können als Handelsprodukt bezogen werden.

Als Oxidationsmittel eignen sich alle Verbindungen, die eine Peroxygruppe -O-O-enthalten.

In Betracht kommen vorzugsweise Peroxodisulfate (auch als Persulfate bezeichnet) der allgemeinen Formel M₂S₂O₈ mit M gleich Ammonium oder Alkalimetall. Bevorzugt ist M gleich Ammonium, Natrium oder Kalium.

Ebenfalls als Oxidationsmittel geeignet sind Peroxide, beispielsweise anorganische Peroxide wie Wasserstoffperoxid oder Metallperoxide M¹₂O₂ (M^{I} gleich einwertiges Metall) wie Na₂O₂ bzw. M^{II}O₂ (M^{II} gleich zweiwertiges Metall) wie BaO₂.

Auch organische Peroxide sind geeignet, beispielsweise
Hydroperoxide R-O-OH,
"echte" Peroxide R-O-O-R wie z.B. Di-tert-butylperoxid oder Dicumylperoxid,
Diacylperoxide R-C(O)-O-O-C(O)-R wie Dibenzoylperoxid oder Diacetylperoxid,
Persäuren R-C(O)-O-OH wie m-Chlorperbenzoesäure,
Persäureester R-C(O)-O-O-R*,
Ketonperoxide wie Aceton-Peroxid und
Epidioxide (Endoperoxide) wie 3,3,4,4-Tetramethyl-1,2-dioxethan,
wobei R bzw. R* Alkyl, Aryl oder Arylalkyl mt 1 bis 30 C-Atomen bedeutet.

Besonders bevorzugt verwendet man ein Peroxodisulfat oder ein Peroxid als Oxidationsmittel. Insbesondere geeignet ist Natriumperoxodisulfat Na₂S₂O₈.

Es versteht sich, dass auch Mischungen verschiedener Oxidationsmittel Anwendung finden können.

Erfindungsgemäß setzt man pro ein Mol Monophenol (der obigen allgemeinen Formel II) insgesamt maximal 0,6 mol Oxidationsmittel ein. Die Untergrenze der Oxidationsmittelmenge richtet sich nach dem gewünschten Umsatz. Bevorzugt werden pro ein Mol Monophenol insgesamt 0,45 bis 0,6 mol, insbesondere 0,49 bis 0,55 mol Oxidationsmittel verwendet. Sofern man Mischungen mehrerer Oxidationsmittel verwendet, beziehen sich die genannten Mengen auf die Summe aller Oxidationsmittel.

Im genannten Stand der Technik US-PS 6 077 979 werden etwa 1 mol Persulfat bzw. Peroxid pro 1 mol 2,4-Dimehtylphenol verwendet, also eine weit überstöchiometrische Menge Oxidationsmittel. Dagegen geht aus den erfindungsgemäßen Mengenangaben hervor, dass man bei der vorliegenden Erfindung eine deutlich geringere, vorzugsweise ungefähr stöchiometrische Oxidationsmittelmenge verwendet, was deutlich bessere Ergebnisse liefert.

Nähere Angaben zur Zugabe des Oxidationsmittels werden weiter unten gemacht.

Üblicherweise wird zusätzlich zum Oxidationsmittel ein Metall oder eine Metallverbindung in einer katalytisch wirksamen Menge mitverwendet. Zwingend erforderlich ist das Metall bzw. die Metallverbindung jedoch nicht. Es eignen sich insbesondere Eisen, Kupfer, und deren Verbindungen. Das Metall kann als solches oder als Legierung verwendet werden. Als Metallverbindungen eignen sich beispielsweise die Halogenide, Sulfate, Nitrate, Phosphate oder Cyanide, z.B. als solche oder in Form ihrer Hydrate.

Besonders bevorzugt verwendet man Eisen oder Eisenverbindungen, insbesondere Eisen(II)-Verbindungen wie Eisen(II)sulfat.

Die Menge des Metalls bzw. der Metallverbindung, sofern mitverwendet, ist üblicherweise nicht kritisch und wird so bemessen, dass das Metall bzw. die Metallverbindung als Katalysator wirken kann. In der Regel beträgt diese Menge 1 bis 20 mol-%, bezogen auf das eingesetzte Monophenol.

Die Oxidationsmittel und das Metall bzw. die Metallverbindung können in an sich bekannter Weise hergestellt werden oder sind handelsüblich, und können als solche, beispielsweise als Feststoff, oder als Lösung bzw. Suspension eingesetzt werden. Als Lösungsmittel für das Oxidationsmittel und ggf. Metall(verbindung) sind beispielsweise Wasser oder wassermischbare Verbindungen - etwa für Peroxodisulfate als Oxidationsmittel - gut geeignet.

Die Umsetzung des Monophenols zum Biphenol wird bevorzugt in Lösung oder Suspension durchgeführt. Als Lösungs- bzw. Suspensionsmittel eignen sich bevorzugt polare Flüssigkeiten, beispielsweise Wasser, Isopropanol, Methyl-tert-butylether (MTBE) oder Acetonitril. Wasser ist bevorzugt und wird beispielsweise als deionisiertes oder vollentsalztes Wasser verwendet.

Es wurde gefunden, dass dann besonders gute Ergebnisse erzielt werden, wenn die Menge des eingesetzten Monophenols, bezogen auf die Summe aller Einsatzstoffe, 5 bis 20 Gew.-% beträgt. Zu den Einsatzstoffen zählen beispielsweise das Monophenol und das Oxidationsmittel, und Hilfsstoffe wie z.B. das katalytisch wirksame Metall bzw. die Metallverbindung sowie das Lösungs- bzw. Suspensionsmittel.

Die Reaktionstemperatur beträgt üblicherweise 0 bis 95, bevorzugt 20 bis 70, insbesondere 40 bis 60°C. Der Druck ist üblicherweise nicht kritisch und beträgt 1 mbar bis 100 bar, bevorzugt 0,1 bis 10 bar.

Das erfindungsgemäße Verfahren kann kontinuierlich oder - bevorzugt - diskontinuierlich in jedem üblichen Reaktor durchgeführt werden, wobei es grundsätzlich möglich ist, rückvermischende oder nicht rückvermischende Reaktoren (d.h. Reaktoren mit Rührkessel- oder Rohrreaktor-Verhalten) zu verwenden. Geeignet sind zum Beispiel Rührkessel, Turmreaktoren, Schlaufenreaktoren sowie Rohrreaktoren oder Rohrbündelreaktoren, die einzeln oder als Kaskade betrieben werden können. Vorzugsweise ist der Reaktor ein Rührkessel.

Erfindungsgemäß enthält der Reaktor keine als Stromstörer wirkenden Einbauten, insbesondere keine stationären oder beweglichen Stromstörer, keine als Stromstörer wirkenden, in die Reaktionsmischung eintauchenden Wärmetauscher, oder ähnliche Bauteile. Im Sinne der Erfindung fällt ein ggf. zur besseren Durchmischung des Reaktorinhalts verwendeter Rührer nicht unter die "als Stromstörer wirkenden Einbauten", d.h. erfindungsgemäß kann durchaus ein Rührer verwendet werden, wie dies beim Rührkesselreaktor der Fall ist.

Geeignete Rührer für den Rührkesselreaktor sind z.B. Scheiben-, Impeller-, Kreuzbalken-, Gitter-, Blatt-, Schrägblatt-, Anker-, Schaufel-, Propeller-, MIG-, Inter-MIG- oder Wendelrührer oder andere übliche Bauarten. Bevorzugt werden die Bedingungen, insbesondere die Bauart, Größe und Form des Rührkessels und des Rührers, sowie die Rührerdrehzahl, derart gewählt, dass sich in der Reaktionsmischung bei eingeschaltetem Rührer im Bereich des Rührers eine Thrombe (Luftblase) bildet.

Erfindungsgemäß wird das Oxidationsmittel - d.h. die Gesamtmenge des Oxidationsmittels - innerhalb einer Zeitspanne von 10 min bis 24 Stunden entweder kontinuierlich, oder diskontinuierlich in mehreren Portionen, zudosiert. Bevorzugt beträgt diese Zeitspanne 30 min bis 12 Stunden, insbesondere 2 bis 10 Stunden. Dabei richtet sich die Zeitspanne u.a. nach den Einsatzmengen der Ausgangsstoffe, Art und Menge der mitverwendeten Lösungs- bzw. Suspensionsmittel, Reaktionstemperatur und -druck, und der gewünschten Reaktionsdauer.

Ebenfalls erfindungsgemäß ist die pro Zeiteinheit (z.B. pro Minute oder pro Stunde) zudosierte Oxidationsmittelmenge nicht über die gesamte Zeitspanne konstant, sondern wird variiert. Die nachfolgenden konkreten Zahlenangaben (Stunden, Kilogramm etc.) dienen nur zur Veranschaulichung und sind nicht als einschränkende Angaben zu verstehen:

Bei diskontinuierlicher Zugabe und einer beispielhaften Zugabezeitspanne von insgesamt 8 Stunden kann man z.B. anfangs (t = 0) eine erste Portion von 30 kg Oxidationsmittel zufügen, nach zwei Stunden (t = 2 h) eine zweite Portion von 20 kg, nach weiteren drei Stunden (t = 5 h) eine dritte Portion von 5 kg und nach weiteren drei Stunden (t = 8 h) eine vierte und letzte Portion von 25 kg. D.h. bei diskontinuierlicher Zugabe sind die einzelnen Portionen und/oder deren zeitlicher Abstand nicht alle gleich groß.

Bei kontinuierlicher Zugabe - diese ist bevorzugt - kann die zugefügte Oxidationsmittelmenge, aufgetragen beispielsweise als Oxidationsmittelstrom in [kg/min] oder [mol/min] über die Zeit in [h], beispielsweise entlang einer auf- oder absteigenden Funktion, einer Stufen- oder Treppenfunktion, einer exponentiellen Funktion, einer ein oder mehrere Minima bzw. Maxima aufweisenden Funktion oder einer Funktion, die einer anderen mathematischen Beziehung gehorcht, zudosiert werden, solange nur der Oxidationsmittelstrom an mindestens zwei Zeitpunkten voneinander verschieden ist. Dosiert man beispielsweise entlang einer Stufenfunktion und einer Zugabezeitspanne von insgesamt 8 Stunden, so kann man z.B. in den ersten 2 Stunden das Oxidationsmittel mit 2 kg/h zufügen, danach 4 Stunden lang mit 0,5 kg/h zudosieren und schließlich in den letzten 2 Stunden mit 1,5 kg/h zudosieren.

Auch Mischformen von kontinuierlicher und diskontinuierlicher Zugabe sind möglich. Beispielsweise kann man bei einer Zugabezeitspanne von insgesamt 8 Stunden in den ersten 3 Stunden das Oxidationsmittel kontinuierlich zugeben, danach 2 Stunden lang kein Oxidationsmittel zugeben, und in den letzten 3 Stunden wieder kontinuierlich Oxidationsmittel zugeben, oder auch in den letzten 3 Stunden 2 Portionen diskontinuierlich zugeben.

In einer bevorzugten Ausführungsform ist das Verfahren dadurch gekennzeichnet, dass die pro Zeiteinheit zudosierte Oxidationsmittelmenge zu Beginn der Umsetzung x beträgt, bis ein Umsatz von 20 % erreicht ist, danach y beträgt, bis ein Umsatz von 40 % erreicht ist, und schließlich bei einem Umsatz von über 40 % z beträgt, wobei gilt: x > y und z > y.

Mit anderen Worten ist bevorzugt die zudosierte Menge bei bis zu 20 % Umsatz und bei über 40 % Umsatz höher als zu einem Zeitpunkt dazwischen. Dabei können x und z gleich oder verschieden sein, und die Oxidationsmittelmenge x, y und z kann z.B. als Stoffmenge, Masse, Volumen, Stoffmengenstrom, Massenstrom oder Volumenstrom ausgedrückt werden.

Ebenfalls bevorzugt gilt die Beziehung y ≤ 0,5 x und y ≤ 0,5 z, d.h. bevorzugt beträgt die pro Zeiteinheit zudosierte Oxidationsmittelmenge zu einem Zeitpunkt bei über 20 bis 40 % Umsatz maximal die Hälfte der bei bis 20 % Umsatz zudosierten Menge x, und maximal die Hälfte der bei über 40 % Umsatz zudosierten Menge z.

Vorzugsweise wird die Umsetzung nicht bis zu einem vollständigen Umsatz geführt, sondern bis zu einem Umsatz von maximal 95 %, besonders bevorzugt maximal 90 %, bezogen auf das eingesetzte Monophenol.

Nach dem Ende der Oxidationsmittelzugabe, d.h. nach Ablauf der Zugabezeitspanne, kann man je nach Reaktionstemperatur und Einsatzstoffmengen noch eine gewisse Zeit nachreagieren lassen (bei Rührkesselreaktoren auch als Nachrühren bezeichnet), um die Reaktion bis zum gewünschten Umsatz zu führen.

Anschließend wird die Reaktionsmischung in üblicher Weise auf das erhaltene Biphenol aufgearbeitet. Wird, wie es bevorzugt ist, in polarem, insbesondere wässrigem Medium umgesetzt, wird das Biphenol üblicherweise durch Zugabe eines Extraktionsmittels aus der Reaktionsmischung extrahiert. Als Extraktionsmittel eignen sich insbesondere aliphatische, araliphatische oder aromatische Kohlenwasserstoffe. Geeignet sind z.B. Toluol, Cyclohexan, Methylcyclohexan oder C₅₋₁₀-Alkane wie die Heptane. Bei dieser Extraktion kann die Temperatur falls erforderlich angepasst werden, und man kann ein- oder mehrfach, mit gleichen oder verschiedenen Extraktionsmitteln extrahieren.

Das extrahierte Biphenol kann danach durch übliche Trennverfahren aus der organischen Phase abgetrennt werden. Beispielsweise kann man durch Abkühlen das Biphenol ausfällen und aus der entstandenen Suspension durch Filtration oder andere fest-flüssig-Trennverfahren abtrennen.

Das erfindungsgemäße Verfahren zeichnet sich dadurch aus, dass in vielen Fällen bereits nach einer einzigen Kristallisation und simplem Abfiltrieren, ein Biphenol hoher Reinheit erhalten wird. Eine mehrfache Kristallisation zur Abtrennung von Nebenprodukten oder komplizierte Trennverfahren sind nicht erforderlich.

Erforderlichenfalls kann das abgetrennte Biphenol zur weiteren Reinigung gewaschen werden, z.B. mit den genannten Extraktionsmitteln, oder auf andere Weise von eventuellen Verunreinigungen befreit werden, und schließlich getrocknet werden.

Das erfindungsgemäße Verfahren vermeidet die erwähnten Nachteile des Standes der Technik. Die Ablagerung klebriger Reaktionsprodukte am Rührer und im Reaktor wird wesentlich vermindert, und der Reaktor muss nur noch selten zwecks Reinigung abgestellt werden. Das Wertprodukt Biphenol kann in technisch einfacher Weise durch einmalige Kristallisation und Abfiltrieren und in guter Reinheit isoliert werden. Die Reinheit des erhaltene Biphenols ist auch bei großtechnischer Durchführung der Umsetzung zufriedenstellend.

Die nach dem erfindungsgemäßen Verfahren erhältlichen Biphenole sind ebenfalls Gegenstand der Erfindung, außerdem die eingangs beschriebene Verwendung der Biphenole zur Herstellung phosphorhaltiger Chelatliganden.

### Beispiele:

### Erfindungsgemäßes Beispiel A

Es wurde ein 2 m³-Stahlemail-Rührkessel mit temperierbarer Außenwand verwendet, der keine Stromstörer oder sonstige Einbauten enthielt. Der Kessel war mit einem Schrägblattrührer ausgestattet, der mit 90 Umdrehungen pro Minute betrieben wurde.

Man legte 760 kg vollentsalztes Wasser vor, gab 13,9 kg (50 mol) Eisen(II)-sulfat-Hepathydrat zu und erwärmte die Lösung auf eine Reaktionstemperatur von 50°C. Nach Zugabe von 122 kg (1000 mol) 2,4-Dimethylphenol dosierte man insgesamt. 619 kg einer 19 gew.-%igen Na₂S₂O₈-Lösung (= 500 mol Na₂S₂O₈) wie folgt zu:
201 kg Lösung in 1 Stunde, entsprechend 3,35 kg Lösung pro Minute, danach
210 kg Lösung in 4 Stunden, entsprechend 0,88 kg Lösung pro Minute, und schließlich
208 kg Lösung in 1 Stunde, entsprechend 3,47 kg Lösung pro Minute.

Nach Dosierende wurde noch 1 Stunde bei 50°C nachgerührt, anschließend auf 70°C erwärmt und 98 kg Toluol zugefügt. Man ließ 600 kg der wässrigen Phase aus dem Reaktor ab, gab zur verbliebenen Toluol-Wasserphase-Mischung bei 70°C 300 kg n-Heptan und ließ auch die restliche wässrige Phase ab. Die im Kessel verbliebene organische Phase wurde mit einer Abkühlrate von 10°C pro Stunde auf 20°C abgekühlt. Man erhielt eine Suspension, die über einen Seitz-Filter filtriert wurde. Der Filterkuchen wurde einmal mit 60 kg n-Heptan gewaschen und auf Trockenblechen bei 50°C im Vakuum-Trockenschrank getrocknet

Die Tabelle gibt die Ausbeute an erhaltenem 3,3'5,5'-Tetramethyl-2,2'-biphenol und die gaschromatographisch mit internem Standard bestimmte Produktreinheit an.

### Vergleichsbeispiel B-V

wurde in Anlehnung an Example 1 in Spalte 2 Zeilen 32-47 der US-PS 6 077 979 durchgeführt. Example 1 wurde vom dort verwendeten 5 I-Laborkolben auf den 2m³-Rührkessel übertragen; dies entsprach einem Scaleup-Faktor 1000 bezüglich 2,4-Dimethylphenol.

Es wurde vorgegangen wie im erfindungsgemäßen Beispiel A dieser Anmeldung beschrieben, mit folgenden Unterschieden:
- die Reaktionstemperatur betrug 25°C,
- es wurden 672 kg einer 35 gew.-%igen Na₂S₂O₈-Lösung zugefügt (= 1000 mol Na₂S₂O₈),
- die Na₂S₂O₈-Lösung wurde innerhalb von 4 Stunden mit konstant 2,8 kg pro Stunde zudosiert,
- es wurde 72 Stunden bei 25°C nachgerührt,
- das Produkt musste zweimal umkristallisiert werden.

### Vergleichsbeispiel C-V

Es wurde vorgegangen wie im Beispiel B-V beschrieben, jedoch enthielt der Rührkessel eingebaute Stromstörer. Am Rührer und im Bereich hinter den Stromstörern lagerte sich ein klebriger Feststoff ab, der nach Leerung des Kessels entfernt werden musste.

**Tabelle: Ergebnisse (V zum Vergleich)**

| Beispiel | Ausbeute *) | Reinheit |
|---|---|---|
| A | 74kg=61 % | >98% |
| B-V | 55 kg = 45 % | 95 % |
| C-V | Versuch abgebrochen | Versuch abgebrochen |

| | | |
|---|---|---|
| *) % sind % der Theorie | | |

Die Beispiele zeigen, dass bei dem erfindungsgemäßen Verfahren (Beispiel A) sowohl die Ausbeute als auch die Produktreinheit deutlich höher waren als bei dem nicht erfindungsgemäßem Vorgehen der Beispiele B-V (Oxidationsmittelüberschuss und konstante Zugabe) bzw. C-V (mit Stromstörern). Diese Vorteile konnten bei wesentlich kürzerer Reaktionsdauer erzielt werden: die Nachreaktionszeit betrug in Beispiel A 1 Stunde statt in Beispiel B-V 72 Stunden.

Im Vergleichsbeispiel B-V wurde die Reinheit von 95 % erst nach zweimaligem Umkristallisieren erreicht, und die Ausbeute war mit 45 % gering. Hingegen betrug im erfindungsgemäßen Beispiel A die Reinheit ohne Umkristallisieren über 98 % bei 61 % Ausbeute.

## Patentansprüche

1. Verfahren zur Herstellung von Biphenolen der allgemeinen Formel I 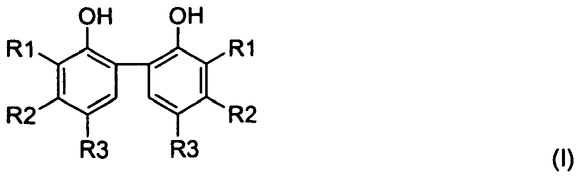 durch Umsetzung von Monophenolen der allgemeinen Formel II 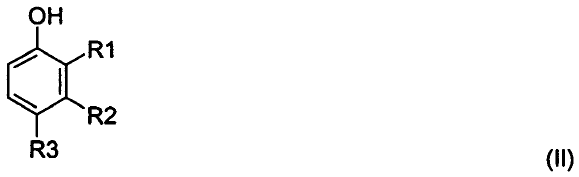 wobei die Reste R1, R2 und R3 unabhängig voneinander Wasserstoff, Alkyl, Aryl oder Arylalkyl mit 1 bis 10 C-Atomen bedeuten, in Gegenwart eines Oxidationsmittels in einem Reaktor, **dadurch gekennzeichnet, dass**
a) der Reaktor keine als Stromstörer wirkenden stationären Einbauten enthält,
b) pro ein Mol Monophenol insgesamt maximal 0,6 Mol Oxidationsmittel eingesetzt werden, und
c) das Oxidationsmittel innerhalb einer Zeitspanne von 10 min bis 24 Stunden entweder kontinuierlich, oder diskontinuierlich in mehreren Portionen, zudosiert wird, wobei die pro Zeiteinheit zudosierte Oxidationsmittelmenge nicht über die gesamte Zeitspanne konstant ist, sondern variiert wird.

2. Verfahren nach Anspruch 1, **dadurch gekennzeichnet, dass** zwei der Reste R1, R2 und R3 Methyl und ein Rest Wasserstoff bedeuten.

3. Verfahren nach den Ansprüchen 1 bis 2, **dadurch gekennzeichnet, dass** das Monophenol 2,4-Dimethylphenol und das Biphenol 3,3',5,5'-Tetramethyl-2,2'-biphenol ist.

4. Verfahren nach den Ansprüchen 1 bis 3, **dadurch gekennzeichnet, dass** das Oxidationsmittel ein Peroxodisulfat oder ein Peroxid ist.

5. Verfahren nach den Ansprüchen 1 bis 4, **dadurch gekennzeichnet, dass** der Reaktor ein Rührkessel ist.

6. Verfahren nach den Ansprüchen 1 bis 5, **dadurch gekennzeichnet, dass** die Menge des eingesetzten Monophenols, bezogen auf die Summe aller Einsatzstoffe, 5 bis 20 Gew.-% beträgt.

7. Verfahren nach den Ansprüchen 1 bis 6, **dadurch gekennzeichnet, dass** pro ein Mol Monophenol insgesamt 0,45 bis 0,6 Mol Oxidationsmittel eingesetzt werden.

8. Verfahren nach den Ansprüchen 1 bis 7, **dadurch gekennzeichnet, dass** die pro Zeiteinheit zudosierte Oxidationsmittelmenge zu Beginn der Umsetzung x beträgt, bis ein Umsatz von 20 % erreicht ist, danach y beträgt, bis ein Umsatz von 40 % erreicht ist, und schließlich bei einem Umsatz von über 40 % z beträgt, wobei gilt: x > y und z > y.

9. Verfahren nach den Ansprüchen 1 bis 8, **dadurch gekennzeichnet, dass** gilt: y ≤ 0,5 x und y ≤ 0,5 z.

10. Verfahren nach den Ansprüchen 1 bis 9, **dadurch gekennzeichnet, dass** die Umsetzung bis zu einem Umsatz von maximal 95 % geführt wird.

11. Verfahren nach den Ansprüchen 1 bis 10, **dadurch gekennzeichnet, dass** die Umsetzung bei einer Temperatur von 40 bis 60°C erfolgt.

## Claims

1. A process for prepaying biphenols of the general formula I 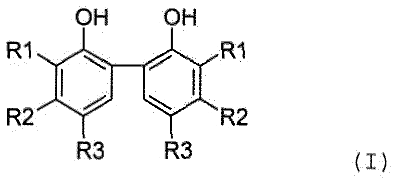 by redaction of monophenols of the general formula II 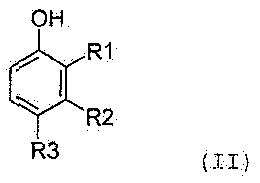 where the radicals R1, R2 and R3 are each, independently of one another, hydrogen, alkyl, aryl or arylalkyl heaving from 1 to 10 carbon atoms, in the presence of an oxidant in a reactor, wherein
a) the reactor comprises no stationary internals which act as baffles,
b) a total of not more than 0.6 mol of oxidant is used per one mol of Monophenol, and
c) the oxidant is introduced either contiguously or discontinuously in a plurality of potions over a period of from 10 minutes to 24 hours, with the amount of oxidant introduced per unit time not bering constant over the total period of time but instead bering varied.

2. The process according to claim 1, wherein two of the radicals R1, R2 and R3 are methyl and once radical is hydrogen.

3. The process according to claim 1 or 2, wherein the Monophenol is 2,4-dimethylphenol and the Biphenol is 3,3',5,5'-tetramethyl-2,2'-biphenol.

4. The process according to any of claims 1 to 3, wherein the oxidant is a peroxodisulfate or a peroxide.

5. The process according to any of claims 1 to 4, wherein the reactor is a stirred vessel.

6. The process according to any of claims 1 to 5, wherein the amount of monophenol used, based on the sum of all starting materials, is from 5 to 20% by weight.

7. The process according to any of claims 1 to 6, wherein a total of from 0.45 to 0.6 mole of oxidant is used per one mole of monophenol.

8. The process according to any of claims 1 to 7, wherein the amount of oxidant introduced per unit time is x at the beginning of the redaction until a conversion of 20% has been reached, then is y until a conversion of 40% has been reached and finally is z at a conversion of above 40%, where: x > y and z > y.

9. The process according to any of claims 1 to 8, wherein:
y ≤ 0.5 x and y ≤ 0.5 z.

10. The process according to any of claims 1 to 9, wherein the redaction is carried out to a conversion of not more than 95%.

11. The process according to any of claims 1 to 10, wherein the reaction is carried out at a temperature of from 40 to 60°C.

## Revendications

1. Procédé de fabrication de biphénols de formule générale I 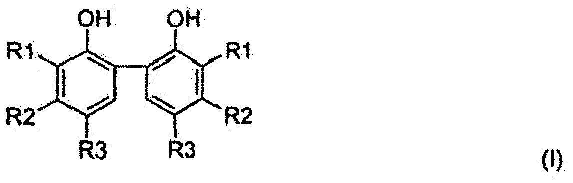 par mise en réaction de monophénols de formule générale II 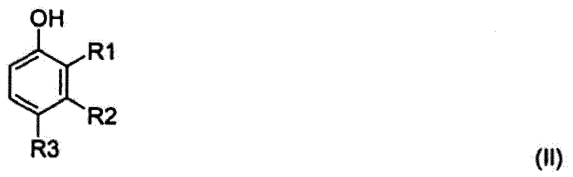 dans lesquelles les radicaux R1, R2 et R3 signifient indépendamment les uns des autres hydrogène, alkyle, aryle ou arylalkyle de 1 à 10 atomes C, en présence d'un oxydant dans un réacteur, **caractérisé en ce que**
a) le réacteur ne contient aucun composant interne stationnaire agissant en tant que déflecteur,
b) au total au plus 0,6 mole d'oxydant est utilisé par mole de monophénol, et
c) l'oxydant est introduit de manière continue ou discontinue, en plusieurs portions, en une durée de 10 min à 24 heures, la quantité d'oxydant introduire par unité de temps n'étant pas constante sur la durée totale, mais étant plutôt variée;

2. Procédé selon la revendication 1, **caractérisé en ce que** deux des radicaux R1, R2 et R3 signifient méthyle et un radical hydrogène.

3. Procédé selon les revendications 1 à 2, **caractérisé en ce que** le monophénol est le 2,4-diméthylphénol et le biphénol est le 3,3',5,5'-tétraméthyl-2,2'-biphénol.

4. Procédé selon les revendication 1 à 3, **caractérisé en ce que** l'oxydant est on peroxodisulfate ou un peroxyde.

5. Procédé selon les revendications 1 à 4, **caractérisé en ce que** le réacteur est une cuve agitée.

6. Procédé selon les revendications 1 à 5, **caractérisé en ce que** la quantité de monophénol utilisé, par rapport à la somme de toutes les matières premières, est de 5 à 20 % en poids.

7. Procédé selon les revendication 1 à 6, **caractérisé en ce qu'**au total 0,45 à 0,6 mole d'oxydant est utilisé par mole de monophénol.

8. Procédé selon les revendications 1 à 7, **caractérisé en ce que** la quantité d'oxydant introduite par unité de temps au début de la réaction est nommée x jusqu'à ce qu'une conversion de 20 % soit atteinte, puis y jusqu'à ce qu'une conversion de 40 % soit atteinte, et enfin z à une conversion supérieure à 40 %, avec : x > y et z > y.

9. Procédé selon les revendications 1 à 8, **caractérisé en ce que** y ≤ 0,5 x et y ≤ 0,5 z.

10. Procédé selon les revendications 1 à 9, **caractérisé en ce que** la réaction est réalisés jusqu'à une conversion d'au plus 95 %.

11. Procédé selon les revendications 1 à 10, **caractérisé en ce que** la réaction a lieu à une température de 40 à 60 °C.
